# EUROPEAN PATENT SPECIFICATION

(11) **EP 2 829 560 B1**
(45) Date of publication and mention of the grant of the patent: **23.03.2016**
(21) Application number: 13188326.6
(22) Date of filing: 11.10.2013
(51) Int. Cl.: C08F 220/58, G01T 1/04

(54) **Polymerizable composition, method of making and using it and 3d polymer gel dosimeter comprising the composition**
Polymerisierbare Zusammensetzung, Verfahren zur Herstellung und Verwendung davon und 3D-Polymergeldosimeter mit der Zusammensetzung
Composition polymérisable, procédé de fabrication et d'utilisation de celle-ci et dosimètre de gel polymère 3D comprenant la composition

(30) Priority: 24.07.2013 US 201313950171
(43) Date of publication of application: 28.01.2015
(73) Proprietor: King Abdulaziz City for Science and Technology, 11442 Riyadh (SA); King Faisal Specialist Hospital & Research Centre, 11211 Riyadh (SA)
(72) Inventor: Basfar, Ahmed Ali, 11442 Riyadh (SA); Moftah, Belal, 11211 Riyadh (SA); Khalil, Salah Lotfy Ahmed, 11331 Cairo (EG); Almousa, Akram, 11211 Riyadh (SA)
(74) Representative: Gulde & Partner

(56) References cited:
- EP-A1- 2 351 779
- US-A- 5 321 357

## Description

### FIELD OF INVENTION

The present invention describes a novel composition of a three dimensional (3D) polymer, method of making the polymer and using the polymer for radiation treatment planning. More specifically it relates to radiation-induced polymerization of N-(isobutoxymethyl) acrylamide normoxic 3D polymer gel dosimeters for planning of radiation treatment.

### BACKGROUND

Over the past decade there has been a growing interest in the development 3D gel dosimeters to aid in the evaluation of the distribution and magnitude of absorbed dose in clinical radiation therapy. The most widely used dosimeter for verification of spatial dose distributions is the polyacrylamide gel (PAG) dosimeter (Fuxman AM, et al. 2005).

Schreiner et al. (2010) introduced polymer gels that are used as chemical dosimeters based on dose dependent radiation-induced polymerization and cross-linking of monomers in an irradiated volume. The changes were spatially localized in the volume by incorporating the initial monomers in an aqueous gel matrix in the dosimeter and can be probed by various imaging techniques such as magnetic resonance imaging (MRI), x-ray computed tomography (CT), and optical CT. As they are chemical dosimeters, polymer gels are sensitive to preparation conditions. The three dimensional dose readout is sensitive to the imaging modality and also to the technical conditions in use during specific scans.

Polymer gel dosimeters offer a wide range of potential applications in the three-dimensional verification of complex dose distribution such as in intensity-modulated radiotherapy (IMRT). However, polymer gel dosimeters have not been widely used in the clinic. One of the reasons is that they are difficult to manufacture. As the polymerization in polymer gels is inhibited by oxygen, all free oxygen has to be removed from the gels. For several years this was achieved by bubbling nitrogen through the gel solutions and by filling the phantoms in a glove box that is perfused with nitrogen. Recently another gel formulation was proposed in which oxygen is bound in a metallo-organic complex, thus removing the problem of oxygen inhibition. The proposed gel consists of methacrylic acid, gelatin, ascorbic acid, hydroquinone and copper (II)sulphate and is given the acronym MAGIC gel dosimeter. These gels are fabricated under normal atmospheric conditions and are therefore called 'normoxic' gel dosimeters. A chemical analysis on the MAGIC gel was performed by Deene and YD (2002). The composition of the gel was varied and its radiation response was evaluated. The role of different chemicals and the reaction kinetics were discussed. It was found that ascorbic acid alone was able to bind the oxygen and can thus be used as an anti-oxidant in a polymer gel dosimeter. It was also found that the anti-oxidants N-acetyl-cysteine and tetrakis(hydroxymethyl)phosphonium were effective in scavenging the oxygen. However, the rate of oxygen scavenging is dependent on the anti-oxidant and its concentration with tetrakis(hydroxymethyl)phosphonium being the most reactive anti-oxidant. Potentiometric oxygen measurements in solution provide an easy way to get a first impression on the rate of oxygen scavenging. It is shown that copper (II) sulphate operates as a catalyst in the oxidation of ascorbic acid; therefore the study proposes some new normoxic gel formulations that have a less complicated chemical formulation than the MAGIC gel. The important factor that can limit the wider use of MAGIC gel dosimeter is temperature, as gel melting can destroy 3D information.

The intra- and inter-batch accuracy and precision of MRI (polyacrylamide gelatin gel fabricated at atmospheric conditions)(PAGAT) polymer gel dosimeters was assessed in full 3D by Jan Vandecasteele et al. (2013). The intra-batch study showed high dosimetric precision (3.1%) notwithstanding poor accuracy (mean dose discrepancies up to 13.0%). In the inter-batch study, a similar dosimetric precision (4.3%) and accuracy (mean dose discrepancies up to 13.7%) were found. The poor dosimetric accuracy was attributed to a systematic fault that was related to the calibration method. Therefore, the dose maps were renormalized using an independent ion chamber dose measurement. It is illustrated that with this renormalization, excellent agreement between the gel measured and TPS calculated 3D dose maps is achievable: 97% and 99% of the pixels meet the 3%/3 mm criteria for the intra- and inter-batch experiments, respectively.

Three new polymer gel dosimeter recipes were investigated by R. J. Senden Pdjkbmaljs (2006). These may be more suitable for widespread applications than polyacrylamide gel dosimeters, since the extremely toxic acrylamide was replaced with the less harmful monomers including N-isopropylacrylamide (NIPAM), diacetone acrylamide and N-vinylformamide. The new gel dosimeters contained gelatin (5 wt%), monomer (3 wt%), N,N'-methylene-bis-acrylamide crosslinker (3 wt%) and tetrakis (hydroxymethyl) phosphonium chloride as antioxidant (10 mM). The NMR response (R₂) of the dosimeters was analyzed for conditions of varying doses, dose rate, time post-irradiation, and temperature during irradiation and scanning. It was shown that the dose-response behavior of the NIPAM/Bis gel dosimeter is comparable to that of normoxic polyacrylamide gel (PAGAT) in terms of high dose-sensitivity and low dependence on dose rate and irradiation temperature, within the ranges considered. The dose-response (R₂) of NIPAM/Bis appears to be linear over a greater dose range (up to 15 Gy) than the PAGAT gel dosimeter. The effects of time post-irradiation (temporal instability) and temperature during NMR scanning on the R₂ response were more significant for NIPAM/Bis dosimeters.

Radiation sensitive gels have been used as dosimeters for clinical dose verification of different radiation therapy modalities. However, the use of gels is not widespread, because careful techniques are required to achieve the dose precision and accuracy aimed for in clinical dose verification. Crescentira et. al.( 2007) introduces a gel dosimetry in a clinical environment is described, including the whole chain of customizations and preparations required to introduce magnetic resonance (MR) based gel dosimetry into clinical routine. In order to standardize gel dosimetry in dose verifications for radiosurgery and intensity modulated radiotherapy (IMRT), customization of the gel composition and the MR imaging parameters to increase its precision was addressed. The relative amount of the components of the normoxic, methacrylic acid based gel (MAGIC) was changed to obtain linear and steep dose response relationship. MR imaging parameters were customized for the different dose ranges used in order to lower the relative standard deviation of the measured transversal relaxation rate (*R*₂). An optimization parameter was introduced to quantify the change in the relative standard deviation of *R*₂ (σ_{R2,rel}) taking the increase in MR time into account. A 9% methacrylic acid gel customized for radiosurgery was found to give a linear dose response up to 40 Gy with a slope of 0.94 Gy⁻¹ s⁻¹, while a 6% methacrylic acid gel customized for IMRT had a linear range up to 3 Gy with a slope of 1.86 Gy⁻¹ s⁻¹. With the help of an introduced optimization parameter, the mean σ_{R2}, really was improved by 13% for the high doses and by 55% for low doses, without increasing MR time to unacceptable values. A mean dose resolution of less than 0.13 Gy has been achieved with the gel and imaging parameters customized for IMRT and a dose resolution from 0.97 Gy (at 5 Gy) to 2.15 Gy(at 40 Gy) for the radiosurgery dose range. While high dose precision was achieved, further work is required to achieve clinically acceptable dose accuracy.

Vandecasteele et. al.(2013) quantified some major physico-chemical factors that influence the validity of MRI (PAGAT) polymer gel dosimetry: temperature history (pre-, during and post-irradiation), oxygen exposure (post-irradiation) and volumetric effects (experiment with phantom in which a small test tube is inserted). Results confirm the effects of thermal history prior to irradiation. By exposing a polymer gel sample to a linear temperature gradient of ∼2.8 °C cm⁻¹ and following the dose deviation as a function of post-irradiation time new insights into temporal variations were added. A clear influence of the temperature treatment on the measured dose distribution is seen during the first hours post-irradiation (resulting in dose deviations up to 12%). This effect diminishes to 5% after 54 hours post-irradiation. Imposing a temperature offset (maximum 6 °C for 3 hours) during and following irradiation on a series of calibration phantoms results in only a small dose deviation of maximum 4%. Surprisingly, oxygen diffusing in a gel dosimeter up to 48 hours post-irradiation was shown to have no effect. However, it is concluded that these physico-chemical effects are important factors that should be addressed to further improve the dosimetric accuracy of 3D MRI polymer gel dosimetry.

A major source of dosimetric inaccuracy in normoxic polymer gel dosimeters has local variations in the concentration of oxygen scavenger. Currently, a phosphorus compound, tetrakis(hydroxymethyl)phosphonium chloride (THPC) as an oxygen scavenger of choice is used in most polymer gel dosimetry studies conducted by Mahbod et.al. (2012). Reactions of THPC in a gel dosimeter are not limited to oxygen. It can possibly be consumed in reacting with gelling agent, water free-radicals and polymer radicals before, during and after irradiation, hence affecting the dose response of the dosimeter in several ways. These reactions are not fully known or understood. Experiments were conducted in an anoxic acrylamide-based gel dosimeter. Scanning electron microscopy results indicate gelatin pores decreasing from 40 to 70 µm and a very different radiation-induced polymer structure in samples containing THPC; Fourier-transform Raman spectroscopy shows a twofold reduction in the dose constants of monomer consumption; however, a significant change in the relative dose constants of monomer consumption as a function of dose could not be detected.

According to Steven Babic (2008), the RPC head phantom and optical CT-scanned FX gels can be used for accurate intensity-modulated radiation therapy dose verification in three dimensions. Radiochromic micelle gel dosimeters seem promising for three-dimensional (3D) radiation dosimetry because they can be read out by optical CT techniques and they have superior spatial stability compared to polymer and Fricke gel dosimeters according to Toljsakbm (2013). However, these are transparent gels and did not change color to indicate the effective treatment dose. Only turbid gels and emulsions with precipitated particles responded to radiation. Their results indicate that the color change was due to the oligomerization within precipitated PCDA crystals, and that liquid-phase emulsified PCDA did not undergo oligomerization. As a result, PCDA is not suitable for use in micelle gel dosimeters, and other radiochromic reporter molecules need to be identified. Unfortunately, all phantoms that were used experienced a color change were turbid and would be unsuitable for 3D dosimetry.

However, none of the above-discussed references discloses or suggests a relatively inexpensive but highly effective 3D polymer composition for dosimetric use. Accordingly, there exists a need in the art to overcome the deficiencies and limitations described herein above.

### SUMMARY

The present invention relates to normoxic polymer gel dosimeters containing N-(isobutoxymethyl) (NIBMA) to make and use as a 3D gel dosimeter and to be used for radiation therapy planning.

A first aspect of the present invention relates to a polymerizable composition for making a 3D gel dosimeter for planning a radiation treatment. The polymerizable composition comprises:
2-5 w/w% of gelatin;
1-2 w/w% ofN-(isobutoxymethyl)acrylamide (NIBMA);
1-4 w/w% ofN,N-methylene-bis-acrylamide (BIS);
0-29 w/w% of at least one co-solvent selected from the group consisting of glycerol, acetone and methanol; and
ultra-pure de-ionized water as a solvent.

According to a preferred embodiment, the composition further comprises 1-20 mM, preferably 5 mM, of trakis(hydroxymethyl)phosphonium chloride (THPC). THPC acts as an anti-oxidant.

The co-solvent is preferably glycerol. In one embodiment, the polymerizable composition comprises 17-20 w/w% of glycerol, preferably 20 w/w%.

An amount of gelatin in the polymerizable composition is preferably 4g w/w%.

In one embodiment, the polymerizable composition comprises 3-4 w/w% of N,N-methylene-bis-acrylamide (BIS), preferably 3 w/w%.

It is further preferred that the polymerizable composition comprises 1-1.8 w/w% of N-(isobutoxymethyl)acrylamide (NIBMA), most preferred 1 w/w%.

The composition according to the present invention includes a mixture of monomers (NIBMA) and crosslinkers (BIS). Due to polymerizable vinyl groups the composition is capable of being polymerized, preferably by exposing it to radiation. For this reason, the composition is referred to as 'polymerizable composition' (or sometimes, not quite correct, as 'polymer composition' or 'polymer gel composition').

In one embodiment, a 3D polymer gel composition comprises of gelatin is between 2-5 g w/w% by weight; N-(isobutoxymethyl)acrylamide (NIBMA) is between 1-2 w/w% by weight; N,N-methylene-bis-acrylamide (BIS) is between 1-4 w/w% by weight; glycerol as co-solvent is between 0-29 w/w% by weight; trakis(hydroxymethyl)phosphonium chloride (THPC) concentration is between 1-20 mM and an ultra-pure de-ionized water as a solvent and at least one of a glycerol, acetone and methanol as co-solvent to make a 3D gel dosimeter for planning a radiation treatment.

In another embodiment, the trakis(hydroxymethyl)phosphonium chloride (THPC) concentration is 5 mM, gelatin is 4 g w/w% by weight, N,N-methylene-bis-acrylamide (BIS) 3 w/w% by weight, N-(isobutoxymethyl)acrylamide (NIBMA) is 1.8 w/w% by weight.

In one embodiment, N-(isobutoxymethyl)acrylamide (NIBMA), a homolog of N-methylolacrylamide (NMA), is the isobutyl ether ofNMA. It contains a readily polymerizable vinyl group as well as a crosslinkable iso-butoxymethyl group.

A second aspect of the present invention relates to a method of making the polymerizable composition according to the invention. The method comprises:
soaking gelatin for preferably 10 minutes in ultra-pure deionized water to make a mixture of the gelatin and deionized water;
heating the mixture of the gelatin and the deionized water for preferably 1 hour at preferably 50 °C to make a gelatin solution;
cooling the gelatin solution to preferably 40°C;
adding N,N-methylene-bis-acrylamide (BIS), N-(isobutoxymethyl)acrylamide (NIBMA) and at least on co-solvent selected from the group consisting of glycerol, acetone and methanol to the gelatin solution and mixing to make a second solution;
cooling the second solution to preferably 35°C;
adding trakis(hydroxymethyl)phosphonium chloride (THPC) to the second solution to make the polymerizable composition (NIBMAGAT); and
storing the polymerizable composition at preferably 10°C in a refrigerator until its use for planning of radiation treatment.

The polymerizable composition according to the invention is preferably a normoxic N-(isobutoxymethyl)acrylamide polymerizable gel. The term 'normoxic' indicates that the composition is fabricated under normal atmospheric conditions. In particular, according to this embodiment the steps of making the gelatin solution, the second solution and the N-(isobutoxymethyl)acrylamide polymerizable composition (NIBMAGAT) to be used for measuring 3D irradiation are performed at normal atmospheric condition.

However, in an alternative embodiment, the method further comprises:
purging a chamber with nitrogen to remove oxygen; and
performing the steps of making the gelatin solution, the second solution and the N-(isobutoxymethyl)acrylamide polymerizable composition (NIBMAGAT) to be used for measuring 3D irradiation results within the purged chamber.

A further aspect of the present invention is related to a method of using the polymerizable composition of the invention, i.e. the 3D polymer gel (NIBMAGAT), for planning a radiation treatment. The method comprises:
making the polymerizable composition (NIBMAGAT) and pouring it into a tube;
storing the tube containing the composition in a refrigerator at 10°C before use; and
irradiating the composition using a linear accelerator at a specific absorbed dose to observe the dose response and plan radiation treatment for clinical use.

Preferably, the absorbed dose is between 0-20 Gy.

In another embodiment, a method of making a normoxic 3D polymer gel (NIBMAGAT) comprises using a combination of chemicals at a certain weight and a ratio and pouring the NIBMAGAT into tubes; storing NIBMAGAT in refrigerator at 10°C before use; and irradiating them using a linear accelerator at a specific absorbed dose to observe the dose response and plan radiation treatment for clinical use. In another embodiment, combination of chemicals is a gelatin, a N-(isobutoxymethyl)acrylamide (NIBMA), a N,N-methylene-bis-acrylamide (BIS), a tetrakis(hydroxymethyl)phosphonium chloride (THPC) and a glycerol. In another embodiment, certain weight and the ratio is the gelatin is between 2-5 g w/w% by weight; the N-(isobutoxymethyl)acrylamide (NIBMA) is between 1-2 w/w% by weight; the N, N -methylene-bis-acrylamide (BIS) is between 1-4 w/w% by weight; the trakis(hydroxymethyl)phosphonium chloride (THPC) concentration is between 1-20 mM and the glycerol as co-solvent is between 0-29 w/w% by weight. In another embodiment, gelatin is 4 wt/wt% by weight, the N,N-methylene-bis-acrylamide (BIS) is 4 wt/wt% by weight, the N-(isobutoxymethyl)acrylamide (NIBMA) is 1 wt/wt% by weight, the trakis(hydroxymethyl)phosphonium chloride (THPC) is 5 mM and the glycerol is 17 wt/wt% by weight and the absorbed dose is between 0-20 Gy.

A further aspect of the present invention is directed to a 3D gel dosimeter comprising the polymerizable composition according to the invention.

The composition and methods disclosed herein may be implemented in any means for achieving various aspects, and may be executed manually or automated using a computer. Other features will be apparent from the accompanying figures and from the detailed description that follows.

### BRIEF DESCRIPTION OF DRAWINGS

Example embodiments are illustrated by way of example in the accompanying figures in which:
Fig. 1 shows dose response curves ofNIBMAGAT (1, 2, 3, and 4) polymer gel for different anti-oxidant concentrations.
Fig. 2 shows variation of absorbance as a function of the absorbed dose for NIBMAGAT (1, 2, 3, and 4) polymer gel for different anti-oxidant concentrations.
Fig. 3 shows dose response curves of NIBMAGAT (5, 6, 7, and 8) polymer gel for different NIBMA concentrations.
Fig. 4 shows dose response curves of NIBMAGAT (5, 6, 7, and 8) polymer gel for different NIBMA concentrations based on 5 and 10 (mM) THPC.
Fig. 5 shows dose response curves ofNIBMAGAT (9, 10, 11, and 12) polymer gel for different BIS concentrations.
Fig. 6 shows relaxation rate for dose response curves of NIBMAGAT (13, 14, 15, and 16) polymer gel for different gelatin concentrations.
Fig. 7 shows dose response curves of NIBMAGAT (13, 14, 15, and 16) polymer gel for different gelatin concentration.
Fig. 8 shows relaxation rate for dose response curves ofNIBMAGAT (17, 18, 19, and 20) polymer gel for different co-solvent.
Fig. 9 shows dose response curves ofNIBMAGAT (21, 22, 23, 24 and 25) polymer gel for different glycerol concentrations.
Fig. 10 shows dose response curves ofNIBMAGAT (21, 22, 23, 24 and 25) polymer gel for different glycerol concentrations.
Fig. 11 shows dose response of NIBMAGAT polymer gel for different glycerol concentrations.
Fig. 12 shows dose response curves of NIBMAGAT (26, 27 and 28) polymer gel for different dose rate under 10 MV radiation energy.
Fig. 13 shows dose response curves ofNIBMAGAT (29, 30 and 31) polymer gel for different radiation energies at 600 Gy/ minute dose rate.
Fig. 14 shows dose response curves of NIBMAGAT (32, 33 and 34) polymer gel as a function of scanning temperature under 10 MV radiation energy at 600 Gy/ minute dose rate.
Fig. 15 shows dose response curves of NIBMAGAT (35, 36, 37 and 34) polymer gel as a function of storage time.
Fig. 16 shows dose responses of NIBMAGAT gels up to 5 Gy under 10 MV radiation energy at a dose rate of 600 Gy/ minute.
Fig. 17 shows dose sensitivity values of NIBMAGAT gel dosimeters for different glycerol concentration.
Fig. 18 shows dose response of NIBMAGAT gels up to 10 Gy for radiation energy of 10 MV at 600 Gy/minute dose rate reproduced 7 times.

Other features of the present embodiments will be apparent from the detailed description and claims that follows.

### DETAILED DESCRIPTION

Several embodiments for 3D polymer gel composition, method of making and the 3D polymer gel to be used in radiation treatment planning are disclosed. Although the present embodiments have been described with reference to specific example embodiments, it will be evident that various modifications and changes may be made to these embodiments without departing from the broader spirit and scope of the various embodiments.

### MATERIALS AND METHODS

The materials gelatin (Type A, bloom 300), N-(isobutoxymethyl)acrylamide (NIBMA), N,N-methylene-bis-acrylamide (BIS), tetrakis(hydroxymethyl) phosphonium chloride (THPC) and glycerol were purchased from Sigma Chemical Co. (St. Louis, Mo, USA). The N-(isobutoxymethyl) acrylamide polymer gels were synthesized under a fume hood in normal atmospheric condition. The NIBMAGAT dosimeters were composed ofN-(isobutoxymethyl)acrylamide (NIBMA) monomer, N,N-methylene-bis-acrylamide (BIS) cross-linker, gelatin (Type A, bloom 300), glycerol and tetrakis(hydroxymethyl) phosphonium chloride (THPC). N-(isobutoxymethyl) acrylamide (NIBMA), a homolog of N-methylolacrylamide (NMA), is the isobutyl ether of NMA. It contains a readily polymerizable vinyl group as well as a crosslinkable iso-butoxymethyl group. The isobutyl group imparts organic solubility to NIBMA permitting the preparation of three general classes of polymers:
a. Organic solvent soluble or solvent based polymers which, on application can be thermoset or crosslinked through either self or external crosslinking mechanisms.
b. Water based or emulsion polymers which, can also be either self or externally crosslinked at the point of application.
c. In radiation curing systems, NIBMA can be used as a reactive diluent.

All of the components present in NIBMA with the exception of a small amount of isobutanol are radiation polymerizable through the vinyl double bond. Upon further heating of the NIBMA-containing radiation-cured polymer, additional crosslinking can take place through the iso-butoxymethyl group. The presence of the iso-butoxymethyl group offers several advantages in emulsion polymers. The organic solubility of NIBMA enhances its compatibility with other vinyl monomers permitting the incorporation of larger quantities into the polymer backbone relative to NMA. The alkyl ether stabilizes the methylol group, thus providing greater resistance to premature crosslinking. The iso-butoxymethyl group in IBMA provides a more controllable cure rate, thus minimizing cracking and checking of the final thermoset polymers. The major polymer properties imparted by NIBMA, but not limited to, include: improved water and solvent resistance, improved adhesion, improved tensile strength, higher impact resistance, flexibility, resistance to blocking and good handing properties.

The method of making the 3D polymer gel is performed by soaking a gelatin between 2-5 g w/w% by weight for 10 minutes in the ultra-pure deionized water to make a mixture of the gelatin and deionized water; heating the mixture of the gelatin and the deionized water for 1 hour at 50 °C to make a gelatin solution; cooling the gelatin solution to 40°C; adding a N,N-methylene-bis-acrylamide (BIS) is between 1-4 w/w% by weight and a N-(isobutoxymethyl) acrylamide (NIBMA) between 1-2 w/w% by weight and a glycerol as co-solvents is between 0-29 w/w% by weight to the gelatin solution and mixing to make a second solution; cooling the second solution to 35°C; and adding a trakis(hydroxymethyl) phosphonium chloride (THPC) between 1-20 mM to the second solution and forming a N-(Isobutoxymethyl)acrylamide (NIBMAGAT) polymer gel and stored at 10°C in a refrigerator until further used for planning of radiation treatment. In order to perform characterization study the polymer gels were filled into 10 mm NMR tube (Wilmad glass, Buena, NJ, USA) and sealed. All gels were stored in a refrigerator (10 °C) overnight prior to irradiation or until further use.

### Irradiation of Polymer Gels:

The irradiation of polymer gels were performed using a 6 MV photon beam of a medical linear accelerator (Varian medical systems, USA) with dose rate of 600 cGy/minute calibrated using ionization chamber. Each sample was filled with gel and was placed in a 30 × 30 × 30 cm³ cubic water phantom. The samples were then irradiated in a beam field of size 10 × 10 cm² with different doses at 5 cm depth and 100 cm (SSD). The sample was transferred back to the refrigerator and kept for about 24 hours before NMR measurements. The dosimeters were irradiated with linear accelerator at absorbed doses up to 30 Gy.

### Nuclear Magnetic Resonance (NMR) Measurement:

The relaxation Rate (*R*₂) measurements were performed using 0.5 Tesla NMR (Bruker, Germany). The main components of a magnetic module are permanent magnetic, correction loop of magnetic field, a magnetic temperature control circuit and a probe and receiver of radio frequency (RF) which consists of an amplifier, magnetic, emitter, filter, preamplifier, receiver, detector and output amplifier. The magnet has 0.5 Tesla of strength. The control module contained three main parts, radio frequency wave transmitter (frequency circuit), and source energy and microprocessor unit. The control module operates by computer control. A standard malti-Spin-Echo Carr Purcell Meiboom Gill (CPMG) sequence was used to measure relaxation time (*T*₂). The irradiated polymer gel sample was put in NMR tube (1 cm diameter and 20 cm height) and lowered into magnetic box (probe head). The 90° pulse was first applied to the spin system that rotates the magnetization down into the x'y' plane. The transverse magnetization begins to diphase. At some point in time after the pulse 90° pulses, a 180° pulse was applied. This pulse rotates the magnetization by 180° about the x-axis. The 180° pulse causes the magnetization to at least partially rephrase and to produce a signal call an echo. The Experiment Supervisor program was chosen to determine *T*₂. The values of relaxation rate (*R*₂ = 1/*T*₂) can be obtained directly from the computer screen. The temperature during measurements was 22 ± 0.5 °C. The nuclear magnetic resonance (NMR) spin-spin relaxation rate (relaxation rate for short form) (R₂) for water proton surrounding polymer formulation was used to investigate the degree of polymerization of NIBMAGAT gels. The change in R₂ corresponding to the degree of polymerization in NIBMAGAT gel increases gradually with absorbed dose up to 20 Gy. Dose response of both gel dosimeters increases with increase of monomer concentration.

### UV-VIS Spectrophotometer:

The absorption spectra of irradiated 3D polymer gel samples in the wavelength range from 350 - 650 nm were measured using UV/VIS spectrophotometer, model Lambda 850, from Perkin-Elmer, USA. A zero Gy vial was inserted in the spectrophotometer prior to every light absorption measurement. Three samples at each absorbed dose were measured, but no significant differences in their characteristics were found during measurements. UV/Vis spectrophotometer was used to investigate the degree of whiteness of irradiated samples of NIBMAGAT which is associated with the degree of polymerization of polymer gel dosimeters. The absorbance increases with absorbed dose for all gel dosimeters in the dose range between 0 and 30 Gy. The absorbance of NIBMAGAT gel significantly increases with increase of THPC concentration, while R₂ of NIBMAGAT gel is slightly affected by increase of THPC concentration, indicating that NMR method is more sensitive to radiation-induced polymerization than UV/Vis spectrophotometer method. It was found that there is no effect of dose rate and radiation energy on NIBMAGAT polymer gel dosimeters. The stability of NIBMAGAT dosimeters after irradiation was up to 8 days.

### Effect of Anti-oxidant Concentrations:

The instant disclosure presents NIBMAGAT polymer gel as a novel normoxic polymer gel dosimeters for radiation therapy with low toxicity, low cost and high dose response. These gels are fabricated under normal atmospheric conditions and are therefore called 'normoxic' gel dosimeters. The effect of anti-oxidant (THPC) concentrations on the response of the NIBMAGAT dosimeter was investigated by preparing different compositions of gel dosimeters as listed in Table 1.

**Table 1: Compositions of NIBMAGAT polymer gels based on 4 % gelatin and 2.5-20 mM THPC.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT-1 | 71.2 | 4 | 1.8 | 3 | 20 | 2.5 |
| NIBMAGAT-2 | 71.2 | 4 | 1.8 | 3 | 20 | 5 |
| NIBMAGAT-3 | 71.2 | 4 | 1.8 | 3 | 20 | 10 |
| NIBMAGAT-4 | 71.2 | 4 | 1.8 | 3 | 20 | 20 |

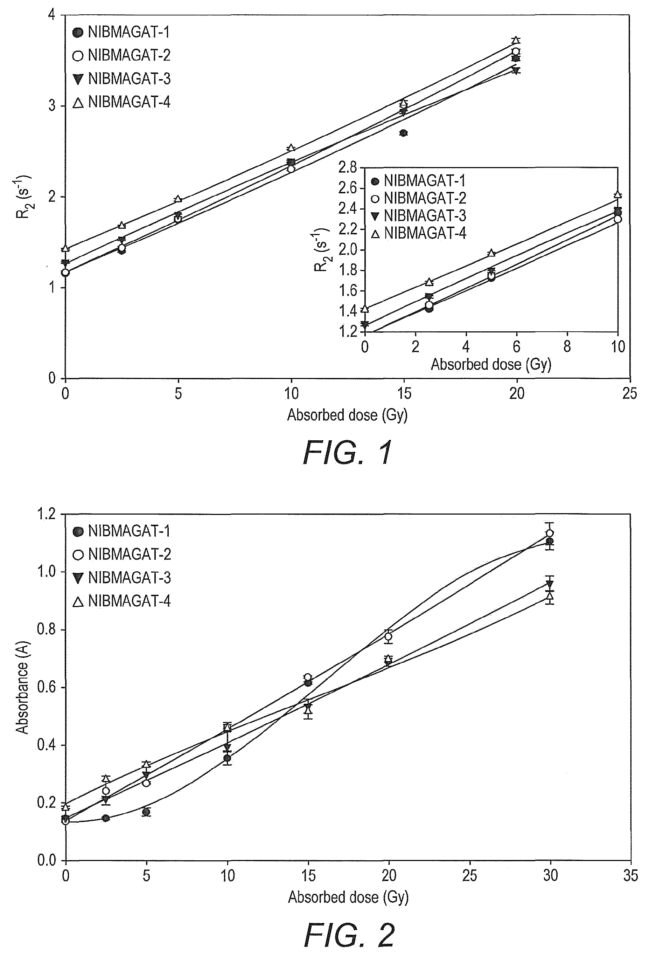

Fig. 1 shows the dose response of different concentrations of THPC in the dose range 2.5-20 Gy. The results show that dose response increases with increase of THPC concentration from 2.5 to 5 mM, but the dose response is not affected with increase of THPC concentration from 5mM to 20 mM, indicating that 5 mM of THPC is sufficient for oxygen scavenging. The results show that dose response increases with increase of THPC concentration, the linearity was observed only for 10 and 20 mM of anti-oxidant concentrations. The results of various concentrations of THPC and NMR relaxation rate show the effect of these chemicals for making optimal 3D NIBMAGAT polymer gels to be used as dosimeter.

**Table 2: NMR Relaxation Rate parameters of NIBMAGAT (1, 2, 3, and 4) polymer gel for different THPC concentrations.**

| THPC Concentration (mM) | Dose, (Gy) | Relaxation Time (T₂), (ms) | Relaxation Rate (R₂ = 1/T2), R₂, (ms⁻¹) | T₂ S.D | Relaxation Rate (R₂ = 1/T₂), R₂,(s⁻¹) | R₂ S.D |
|---|---|---|---|---|---|---|
| 2.5 | 0 | 857.111 | 0.001167 | 3.683 | 1.167 | 0.005015 |
| | 2.5 | 711.667 | 0.001405 | 2.31 | 1.405 | 0.00456 |
| | 5 | 571.6223 | 0.001749 | 3.044 | 1.749 | 0.009314 |
| | 10 | 420.511 | 0.002378 | 2.339 | 2.378 | 0.013227 |
| | 15 | 371.501 | 0.002692 | 2.496 | 2.692 | 0.018087 |
| | 20 | 284.699 | 0.003512 | 2.104 | 3.512 | 0.025955 |
| | | | | | | |
| 5 | 0 | 847.556 | 0.00118 | 4.31 | 1.18 | 0.006001 |
| | 2.5 | 691.444 | 0.001446 | 2.9 | 1.446 | 0.006065 |
| | 5 | 569.0113 | 0.001757 | 2.6 | 1.757 | 0.008028 |
| | 10 | 435.078 | 0.002298 | 2.51 | 2.298 | 0.013257 |
| | 15 | 332.156 | 0.003011 | 2.1 | 3.011 | 0.019037 |
| | 20 | 279.156 | 0.003582 | 2.19 | 3.582 | 0.028101 |
| | | | | | | |
| 10 | 0 | 775.778 | 0.001289 | 3.43 | 1.289 | 0.005699 |
| | 2.5 | 651.667 | 0.001535 | 3.67 | 1.535 | 0.008645 |
| | 5 | 555.556 | 0.0018 | 3.59 | 1.8 | 0.011632 |
| | 10 | 418.322 | 0.002391 | 2.87 | 2.391 | 0.016404 |
| | 15 | 341.256 | 0.00293 | 2.7 | 2.93 | 0.023182 |
| | 20 | 294.978 | 0.00339 | 2.07 | 3.39 | 0.023789 |
| | | | | | | |
| 20 | 0 | 700.776 | 0.001427 | 2.53 | 1.427 | 0.005152 |
| | 2.5 | 596.555 | 0.001676 | 2.36 | 1.676 | 0.00663 |
| | 5 | 508.533 | 0.001966 | 2.68 | 1.966 | 0.010361 |
| | 10 | 395.944 | 0.002526 | 1.85 | 2.526 | 0.011802 |
| | 15 | 330.289 | 0.003028 | 2.45 | 3.028 | 0.022461 |
| | 20 | 321.3 | 0.003112 | 2.71 | 3.112 | 0.026248 |

Fig. 2 Variation of absorbance as a function of the absorbed dose for NIBMAGAT (1, 2, 3, and 4) polymer gel for different anti-oxidant concentrations. The optical absorbance characteristics of the dose response of different concentrations of THPC in the dose range 2.5-20 Gy were measured over the wavelength range from 350 - 650 nm using UV/VIS spectrophotometer. Gel samples NIBMAGAT (1, 2, 3, and 4) polymer gels represent different anti-oxidant concentrations. Blank sample cuvette was mounted in the reference beam. Fig. 2 shows the variation of the absorbance of 3D polymer gels of different concentrations of THPC in the dose range 2.5-20 Gy. The results show that dose response increases with increase of THPC concentration, the linearity was observed only for 10 and 20 mM of anti-oxidant concentrations.

### Effect of NIBMA Concentrations:

The effect of NIBMA concentrations on the response of the NIBMAGAT dosimeter was investigated by preparing different compositions of gel dosimeters as listed in Table 3. The selected THPC concentration is 5 mM. In the instant invention we introduced a monomer N-(isobutoxymethyl)acrylamide (NIBMA) with minimum effective concentration and in normoxic condition when compared to Mahbod et.al (2012).

**Table 3: Compositions of NIBMAGAT polymer gels based on 4 % gelatin, 5 (mM) THPC and 1-2 (w/w%) NIBMA.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT-5 | 72 | 4 | 1 | 3 | 20 | 5 |
| NIBMAGAT-6 | 71.5 | 4 | 1.5 | 3 | 20 | 5 |
| NIBMAGAT-7 | 71.2 | 4 | 1.8 | 3 | 20 | 5 |
| NIBMAGAT-8 | 71 | 4 | 2 | 3 | 20 | 5 |

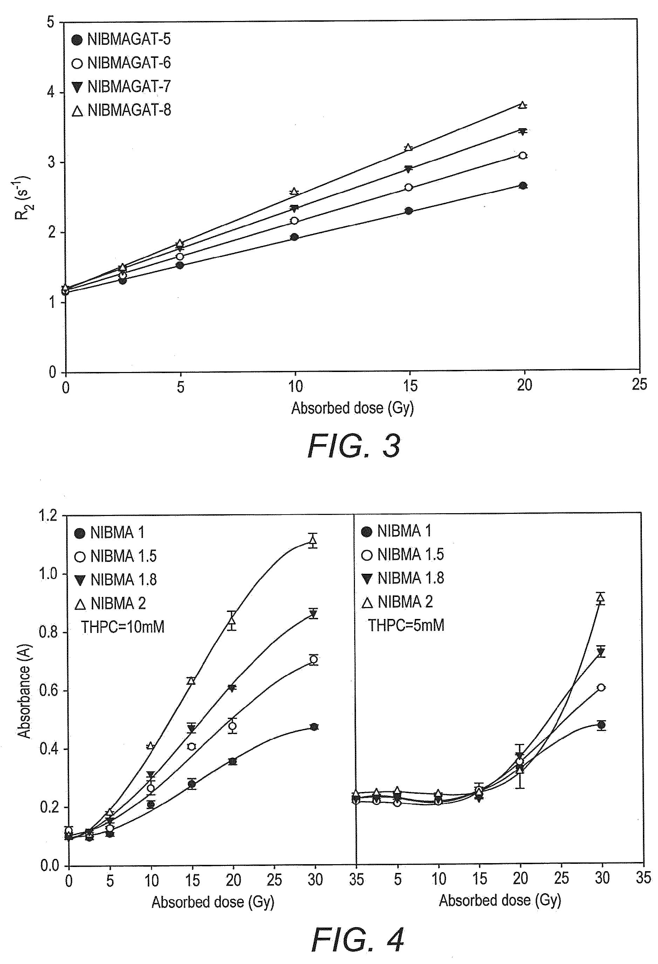

Fig. 3 shows dose response curves of NIBMAGAT (5, 6, 7, and 8) polymer gel for different NIBMA concentrations. The effect of NIBMA concentrations on the response of the NIBMAGAT dosimeter was investigated by preparing different compositions of gel dosimeters as listed in Table 4. The selected THPC concentration is 10 mM. The increase in concentration of monomers increases, dose sensitivity and dose resolution improve. The change in R₂ corresponding to the amount of polymer formation in NIBMAGAT gel increases gradually with absorbed dose up to 20 Gy. The dose response of gel dosimeters increases with increase of monomer concentration.

**Table 4: Compositions of NIBMAGAT polymer gels based on 4 % gelatin, 10 (mM) THPC and 1-2 (w/w %) NIBMA.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT-5 | 72 | 4 | 1 | 3 | 20 | 10 |
| NIBMAGAT-6 | 71.5 | 4 | 1.5 | 3 | 20 | 10 |
| NIBMAGAT-7 | 71.2 | 4 | 1.8 | 3 | 20 | 10 |
| NIBMAGAT-8 | 71 | 4 | 2 | 3 | 20 | 10 |

The sensitivity of polymer gel dosimeters to radiation is directly related to %T, the total weight percent of monomer and THPC concentration in the system. Fig. 4 shows dose response curves of NIBMAGAT (5, 6, 7, and 8) polymer gel for different NIBMA concentrations based on 5 and 10 (mM) THPC.The results are also presented in Table 5 for further clarifications. Fig. 4 also shows the dose response curves of different NIBMA concentrations based on 5 and 10 (mM) THPC in the dose range 2.5-30 Gy. The results show that the dose sensitivity increases significantly with addition of 10 mM THPC.

**Table 5: NMR Relaxation Rate parameters of NIBMAGAT (5, 6, 7, and 8) polymer gel for different NIBMA concentrations.**

| NIBMA concentrations (w/w%) | Dose, (Gy) | Relaxation Time (T₂), (ms) | Relaxation Rate (R₂ = 1/T₂), R₂, (ms⁻¹) | T₂ S.D | Relaxation Rate (R₂ = 1/T₂), R₂,( s⁻¹) | R₂ S.D |
|---|---|---|---|---|---|---|
| 1 | 0 | 872.333 | 0.001146 | 4.27 | 1.146 | 0.00561 |
| | 2.5 | 760.56 | 0.001315 | 4.31 | 1.315 | 0.007452 |
| | 5 | 658.11 | 0.001519 | 4.18 | 1.519 | 0.009648 |
| | 10 | 521.37 | 0.001918 | 2.93 | 1.918 | 0.010779 |
| | 15 | 437.45 | 0.002286 | 1.95 | 2.286 | 0.01019 |
| | 20 | 381.667 | 0.00262 | 2.5 | 2.62 | 0.017162 |
| 1.5 | 0 | 854.333 | 0.001171 | 4.65 | 1.171 | 0.006374 |
| | 2.5 | 724.333 | 0.001381 | 3.22 | 1.381 | 0.006139 |
| | 5 | 608.444 | 0.001644 | 2.91 | 1.644 | 0.007863 |
| | 10 | 464.74 | 0.002152 | 2.89 | 2.152 | 0.013382 |
| | 15 | 381.26 | 0.002623 | 2.73 | 2.623 | 0.018782 |
| | 20 | 327.7333 | 0.003051 | 2.43 | 3.051 | 0.022622 |
| | | | | | | |
| 1.8 | 0 | 831.388 | 0.001203 | 17.1 | 1.203 | 0.024743 |
| | 2.5 | 679.89 | 0.001471 | 4.58 | 1.471 | 0.009909 |
| | 5 | 568.73 | 0.001758 | 3.76 | 1.758 | 0.011623 |
| | 10 | 427.8 | 0.002338 | 3.02 | 2.338 | 0.016505 |
| | 15 | 346.155 | 0.002889 | 2 | 2.889 | 0.016692 |
| | 20 | 293.544 | 0.003407 | 1.79 | 3.407 | 0.020776 |
| | | | | | | |
| 2 | 0 | 838.111 | 0.001193 | 5.47 | 1.193 | 0.007786 |
| | 2.5 | 671.89 | 0.001488 | 3.39 | 1.488 | 0.007508 |
| | 5 | 548.244 | 0.001824 | 2.81 | 1.824 | 0.009349 |
| | 10 | 390.933 | 0.002558 | 2.66 | 2.558 | 0.017405 |
| | 15 | 314.567 | 0.003179 | 1.97 | 3.179 | 0.019909 |
| | 20 | 265.922 | 0.00376 | 1.4 | 3.76 | 0.019795 |

### Effect of BIS Concentrations:

The effect of bis-acrylamide (BIS) concentration on the dose response of the NIBMAGAT dosimeter was investigated by preparing different compositions of gel dosimeters as listed in Table 6.

**Table 6: Compositions of NIBMAGAT polymer gels based on 4 % gelatin and 1-4 (w/w %) BIS.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT-9 | 74 | 4 | 1 | 1 | 20 | 5 |
| NIBMAGAT-10 | 73 | 4 | 1 | 2 | 20 | 5 |
| NIBMAGAT-11 | 72 | 4 | 1 | 3 | 20 | 5 |
| NIBMAGAT-12 | 71 | 4 | 1 | 4 | 20 | 5 |

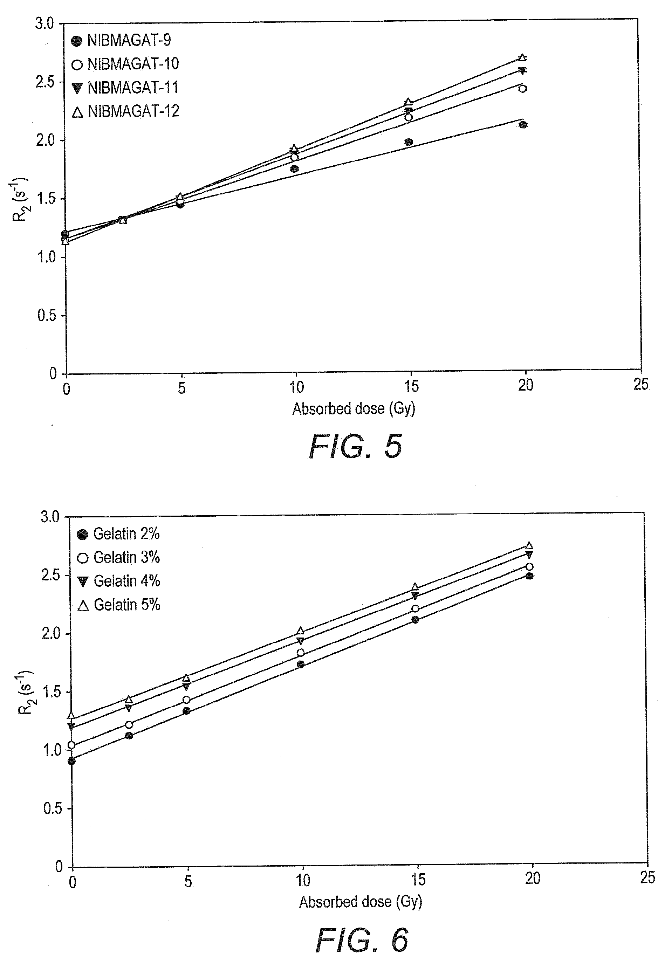

Fig. 5 shows dose response curves ofNIBMAGAT (9, 10, 11, and 12) polymer gel for different BIS concentrations. The change in the proton relaxation rate R₂ versus absorbed dose for polymerization of NHMAGAT for BIS concentration from 1 to 4 wt% is shown in Fig. 5. The dose response of the gel increases gradually with increase in dose. Upon irradiation, water molecules dissociate into OH and Ḣ radicals that break the double C=C bonds of co-monomers (NIBMA and BIS). The resulting co-monomer radicals, in turn, interact with other co-monomers and produce a chain reaction to form 3D polymer aggregates that are spatially retained in a gelatin matrix. The differential rates of monomer and cross-linker consumption, the unreacted monomer/crosslinker ratio varied as gels were irradiated. Free radical polymerization of monomer is kinetically controlled and results in a polymer network with a distribution of different crosslinking densities producing an inhomogeneous network. The model predicts the concentrations of bisacrylamide, pendant double bonds, cyclized groups and cross-link units as well as temperature at different times and positions within the dosimeter, and accounts for the formation of insoluble microgels. When manufactured with glycerol, which is a co-solvent that permits dissolution of additional bisacrylamide above its water solubility. The inhomogeneity of the copolymer network formed is reportedly due to differences in the reactivity of monomeric double bonds with bisacrylamide being more reactive than acrylamide. As a result, the polymer initially formed is rich in bisacrylamide, however, as polymerization proceeds the polymer formed is progressively poorer in bisacrylamide and a heterogeneous structure results. The results are also presented in Table 7 for further clarifications.

**Table 7: NMR Relaxation Rate parameters ofNIBMAGAT (9, 10, 11, and 12) polymer gel for different BIS concentrations.**

| BIS concentrations (w/w%) | Dose, (Gy) | Relaxation Time (T₂), (ms) | Relaxation Rate (R₂ = 1/T₂), R₂, (ms⁻¹) | T₂ S.D | Relaxation Rate (R₂ = 1/T₂), R₂,(s⁻¹) | R₂ S.D |
|---|---|---|---|---|---|---|
| 1 | 0 | 834.445 | 0.001198 | 3.76 | 1.198 | 0.005398 |
| | 2.5 | 759.44 | 0.001317 | 3.87 | 1.317 | 0.006711 |
| | 5 | 689.3556 | 0.001451 | 4.063 | 1.451 | 0.008552 |
| | 10 | 576.122 | 0.001736 | 2.953 | 1.736 | 0.008898 |
| | 15 | 509.778 | 0.001962 | 2.797 | 1.962 | 0.010765 |
| | 20 | 475.633 | 0.002102 | 2.923 | 2.102 | 0.012918 |
| | | | | | | |
| 2 | 0 | 872 | 0.001147 | 2.679 | 1.147 | 0.003524 |
| | 2.5 | 763.889 | 0.001309 | 4.894 | 1.309 | 0.008386 |
| | 5 | 674.911 | 0.001482 | 3.509 | 1.482 | 0.007705 |
| | 10 | 541.889 | 0.001845 | 3.113 | 1.845 | 0.010599 |
| | 15 | 459.567 | 0.002176 | 2.762 | 2.176 | 0.013078 |
| | 20 | 414.8 | 0.002411 | 1.764 | 2.411 | 0.010253 |
| | | | | | | |
| 3 | 0 | 849.999 | 0.001176 | 7.27 | 1.176 | 0.010058 |
| | 2.5 | 758.667 | 0.001318 | 3.519 | 1.318 | 0.006113 |
| | 5 | 668.111 | 0.001497 | 3.719 | 1.497 | 0.008333 |
| | 10 | 530.333 | 0.001886 | 3.34 | 1.886 | 0.011878 |
| | 15 | 447.311 | 0.002236 | 2.482 | 2.236 | 0.012407 |
| | 20 | 389.822 | 0.002565 | 2.219 | 2.565 | 0.014601 |
| | | | | | | |
| 4 | 0 | 880.222 | 0.001136 | 2.474 | 1.136 | 0.003193 |
| | 2.5 | 767.444 | 0.001303 | 3.75 | 1.303 | 0.006367 |
| | 5 | 663.667 | 0.001507 | 2.861 | 1.507 | 0.006497 |
| | 10 | 523 | 0.001912 | 2.403 | 1.912 | 0.008785 |
| | 15 | 435.311 | 0.002297 | 2.868 | 2.297 | 0.015134 |
| | 20 | 374.4 | 0.002671 | 2.138 | 2.671 | 0.015253 |

### Effect of Gelatin Concentrations:

The effect of gelatin concentration on the response of the NIBMAGAT dosimeter was investigated by preparing different compositions of gel dosimeters as listed in Table 8. It may be concluded that THPC not only scavenges radical species but also modifies the morphology of the gelatin network and of the polymer, possibly by intervening in the polymerization of monomers.

**Table 8: Compositions of NIBMAGAT polymer gels based on 1 % NIBMA and 1-4 (w/w %) gelatin.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT- 13 | 74 | 2 | 1 | 3 | 20 | 5 |
| NIBMAGAT- 14 | 73 | 3 | 1 | 3 | 20 | 5 |
| NIBMAGAT- 15 | 72 | 4 | 1 | 3 | 20 | 5 |
| NIBMAGAT-16 | 71 | 5 | 1 | 3 | 20 | 5 |

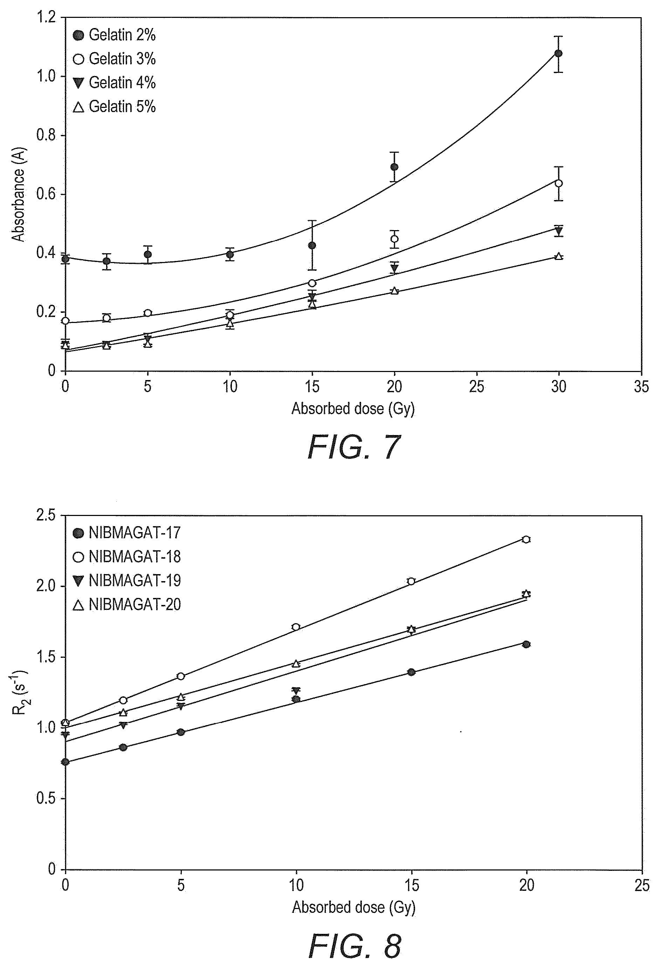

Gelatin was added because, in addition to being soft-tissue equivalent it has low melting point. The low melting point helps prevents dissolved oxygen in the solution which occurs upon heating and enhances the performance. The dose response increases significantly with increase of gelatin concentration from 2 to 5 % within gel dosimeters. The variation of absorbance versus absorbed dose for polymerization of NIBMAGAT having 2 to 5 wt% concentration of gelatin as shown in Fig. 7. Reduced gelatin levels produced significant differences in absorbance. The differences in slopes arising from the different gel with the highest dose sensitivity were obtained for the dosimeter between 3% - 5 % gelatin. This result was somewhat surprising because we anticipated that the dosimeter with the highest gelatin concentration would have the lowest dose sensitivity, for both optical and NMR measurements.

Lower gelatin levels lead to higher optical dose sensitivity. Therefore, when using optical imaging, lowering the gelatin concentration is recommended (along with reduced %T) to produce gels with adequate sensitivity and less light scattering. The spectra of gels prepared with different gelatin concentrations showed little influence of gelatin concentration on dose sensitivity. Fig. 6 shows relaxation rate dose response curves ofNIBMAGAT (13, 14, 15, and 16) polymer gel for different gelatin concentrations.

Fig. 7 shows dose response curves of NIBMAGAT (13, 14, 15, and 16) polymer gel for different gelatin concentrations. The results are also presented in Table 9 for further clarifications.

**Table 9: NMR Relaxation Rate parameters ofNIBMAGAT (13, 14, 15, and 16) polymer gel for different gelatin concentrations.**

| Gelatin concentrations (w/w%) | Dose, (Gy) | Relaxation Time (T₂), (ms) | Relaxation Rate (R₂ = 1/T2), R₂, (ms⁻¹) | T₂ S.D | Relaxation Rate (R₂ = 1/T₂), R₂,(s⁻¹) | R₂ S.D |
|---|---|---|---|---|---|---|
| | 0 | 1095.889 | 0.000913 | 7.545 | 0.913 | 0.006286 |
| | 2.5 | 887.889 | 0.001126 | 3.99 | 1.126 | 0.00506 |
| 2 | 5 | 751.889 | 0.00133 | 3.96 | 1.33 | 0.007005 |
| | 10 | 580.078 | 0.001724 | 3.33 | 1.724 | 0.009897 |
| | 15 | 478.033 | 0.002092 | 2.88 | 2.092 | 0.012604 |
| | 20 | 406.47 | 0.00246 | 2.51 | 2.46 | 0.015191 |
| | | | | | | |
| | 0 | 952.22 | 0.00105 | 3.8 | 1.05 | 0.00419 |
| | 2.5 | 822.89 | 0.001215 | 3.38 | 1.215 | 0.004991 |
| 3 | 5 | 700 | 0.001429 | 3.12 | 1.429 | 0.006369 |
| | 10 | 548.86 | 0.001822 | 3.44 | 1.822 | 0.011419 |
| | 15 | 457.111 | 0.002188 | 2.74 | 2.188 | 0.013115 |
| | 20 | 393.49 | 0.002541 | 2.19 | 2.541 | 0.014142 |
| | | | | | | |
| | 0 | 822.222 | 0.001216 | 3.87 | 1.216 | 0.005723 |
| | 2.5 | 830.556 | 0.001369 | 4.47 | 1.369 | 0.007368 |
| 4 | 5 | 644.833 | 0.001551 | 4.171 | 1.551 | 0.010032 |
| | 10 | 516.9 | 0.001935 | 1.964 | 1.935 | 0.007352 |
| | 15 | 433.644 | 0.002306 | 2.554 | 2.306 | 0.013581 |
| | 20 | 377 | 0.002653 | 1.936 | 2.653 | 0.013624 |
| | | | | | | |
| | 0 | 769.556 | 0.001299 | 3.665 | 1.299 | 0.006186 |
| 5 | 2.5 | 698.889 | 0.001431 | 2.685 | 1.431 | 0.005498 |
| | 5 | 621.855 | 0.001608 | 3.504 | 1.608 | 0.009061 |
| | 10 | 497.844 | 0.002009 | 2.873 | 2.009 | 0.011594 |
| | 15 | 420.411 | 0.002379 | 2.249 | 2.379 | 0.012727 |
| | 20 | 367.5 | 0.002721 | 2.07 | 2.721 | 0.015326 |

### Effect of Co-solvent on NIBMAGAT Dose Response:

The effect of different co-solvents on the response of the NIBMAGAT dosimeter was investigated by preparing different compositions of gel dosimeters as listed in Table 10. As compared to R. J. Senden Pdjkbmaljs (2006). The instant NIBMAGAT dose-response (R₂) appears to be linear over a greater dose range (up to 30 Gy).

**Table 10: Compositions of NIBMAGAT polymer gels based on 4 % Gelatin with different co-solvents.**

| Formulation code | Water (w/w%) | Gelatin (w/w%) | NIBMA (w/w%) | BIS (w/w%) | co-solvent 20 (w/w%) | THPC (mM) |
|---|---|---|---|---|---|---|
| NIBMAGAT- 17 | 92 | 4 | 1 | 3 | Water | 5 |
| NIBMAGAT- 18 | 72 | 4 | 1 | 3 | Glycerol | 5 |
| NIBMAGAT- 19 | 72 | 4 | 1 | 3 | Acetone | 5 |
| NIBMAGAT- 20 | 72 | 4 | 1 | 3 | Methanol | 5 |

Fig. 8 shows the dose response of different co-solvents in the dose range 0 - 20 Gy. The results show that dose response increases in order of water, acetone, methanol and glycerol. There is a significant increase in dose response when organic solvent is used as co-solvent. But, the dose response is not affected by using either acetone or methanol. Another significant dose response increase was found when the glycerol is used as co-solvent. Fig. 8 shows dose response curves of NIBMAGAT (17, 18, 19, and 20) polymer gel for different co-solvent. The results are also presented in Table 11 for further clarifications.

**Table 11: NMR Relaxation Rate parameters ofNIBMAGAT (17, 18, 19, and 20) polymer gel for different co-solvents.**

| Co-Solvent | Dose, (Gy) | Relaxation Time (T₂), (ms) | Relaxation Rate (R₂ = 1/T2), R₂, (ms⁻¹) | T₂ S.D | Relaxation Rate (R₂ = 1/T₂), R₂(s⁻¹) | R₂ S.D |
|---|---|---|---|---|---|---|
| | 0 | 1319.56 | 0.000758 | 7.61 | 0.758 | 0.004371 |
| | 2.5 | 1156 | 0.000865 | 6.227 | 0.865 | 0.004659 |
| Water | 5 | 1025.778 | 0.000975 | 4.63 | 0.975 | 0.004401 |
| | 10 | 828.333 | 0.001207 | 4.187 | 1.207 | 0.006101 |
| | 15 | 715.444 | 0.001398 | 3.69 | 1.398 | 0.00721 |
| | 20 | 627.778 | 0.001593 | 3.377 | 1.593 | 0.008569 |
| | | | | | | |
| | 0 | 961.445 | 0.00104 | 5.01 | 1.04 | 0.005419 |
| | 2.5 | 835.778 | 0.001196 | 3.313 | 1.196 | 0.004741 |
| Glycerol | 5 | 729.667 | 0.00137 | 3.535 | 1.37 | 0.006637 |
| | 10 | 583.444 | 0.001714 | 2.698 | 1.714 | 0.007926 |
| | 15 | 490.333 | 0.002039 | 2.857 | 2.039 | 0.011881 |
| | 20 | 429.111 | 0.00233 | 2.242 | 2.33 | 0.012174 |
| | | | | | | |
| | 0 | 1040.444 | 0.000961 | 8.5 | 0.961 | 0.007851 |
| | 2.5 | 970.667 | 0.00103 | 4.41 | 1.03 | 0.00468 |
| Acetone | 5 | 857.889 | 0.001166 | 4.973 | 1.166 | 0.006759 |
| | 10 | 784.778 | 0.001274 | 3.756 | 1.274 | 0.006097 |
| | 15 | 588.778 | 0.001698 | 2.749 | 1.698 | 0.007928 |
| | 20 | 514.222 | 0.001945 | 2.262 | 1.945 | 0.008556 |
| | | | | | | |
| | 0 | 963.667 | 0.001038 | 6.28 | 1.038 | 0.006764 |
| | 2.5 | 903.667 | 0.001107 | 4.93 | 1.107 | 0.006039 |
| Methanol | 5 | 824.556 | 0.001213 | 4.686 | 1.213 | 0.006894 |
| | 10 | 690.778 | 0.001448 | 2.55 | 1.448 | 0.005345 |
| | 15 | 589.44 | 0.001697 | 2.875 | 1.697 | 0.008277 |
| | 20 | 513.555 | 0.001947 | 2.199 | 1.947 | 0.008337 |

### Effect of Glycerol Concentrations:

The effect of glycerol concentration on the response of the NIBMAGAT dosimeter was investigated by preparing different compositions of gel dosimeters as listed in Table 12.

**Table 12: Compositions of NIBMAGAT polymer gels based on 4 % gelatin 0-29 (w/w %) glycerol.**

| Formulation code | Water (w/w%) | Gelatin (w/w%) | NIBMA (w/w%) | BIS (w/w%) | Glycerol (w/w%) | THPC (mM) |
|---|---|---|---|---|---|---|
| NIBMAGAT-21 | 92 | 4 | 1 | 3 | 0 | 5 |
| NIBMAGAT- 22 | 83 | 4 | 1 | 3 | 9 | 5 |
| NIBMAGAT- 23 | 75 | 4 | 1 | 3 | 17 | 5 |
| NIBMAGAT- 24 | 69 | 4 | 1 | 3 | 23 | 5 |
| NIBMAGAT- 25 | 63 | 4 | 1 | 3 | 29 | 5 |

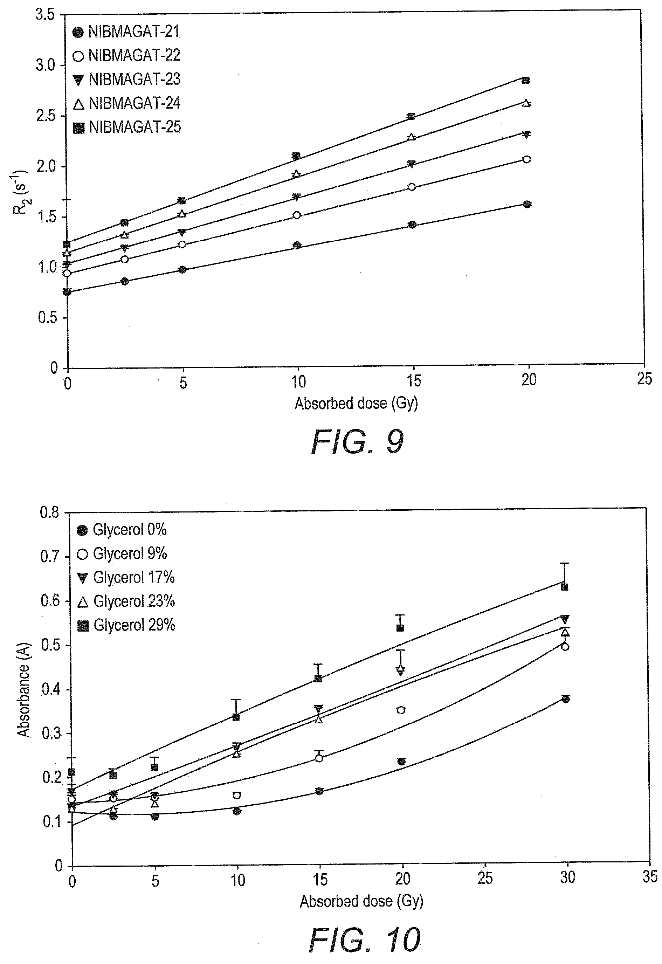

Fig. 9 illustrates the relaxation rate response curves of NIBMAGAT (21, 22, 23, 24 and 25) polymer gel for different glycerol concentrations in the region 0 - 20 Gy. The addition of glycerol, even as low as 17% concentration, results in increased NMR response sensitivity. This effect increases with the amount of co-solvent up to a limit of ≈30% of initial glycerol concentration. The dose response increases significantly with increase of glycerol concentration up to ≈ 30 % as shown in Fig. 9.

Addition of co-solvent increases the relative rate of consumption of bisacrylamide as well as being good solvent for NIBMA monomer, since NIBMA consumption rates remain unchanged with the addition of co-solvent. The results show that the solubility of NIBMA within NIBMAGAT polymer gels increases with increasing glycerol concentration. The results are also presented in table 13 for further clarifications.

**Table 13: NMR Relaxation Rate parameters of NIBMAGAT (21, 22, 23, 24 and 25) polymer gel for different glycerol concentrations**

| Glycerol concentrations (w/w%) | Dose, (Gy) | Relaxation Time (T₂), (ms) | Relaxation Rate (R₂ = 1/T₂), R₂, (ms⁻¹) | T₂ S.D. | Relaxation Rate (R₂ = 1/T₂), R₂,(s⁻¹) | R₂ S.D. |
|---|---|---|---|---|---|---|
| 0 | 0 | 1319.56 | 0.000758 | 7.61 | 0.758 | 0.004371 |
| | 2.5 | 1156 | 0.000865 | 6.227 | 0.865 | 0.004659 |
| | 5 | 1025.778 | 0.000975 | 4.63 | 0.975 | 0.004401 |
| | 10 | 828.333 | 0.001207 | 4.187 | 1.207 | 0.006101 |
| | 15 | 715.444 | 0.001398 | 3.69 | 1.398 | 0.00721 |
| | 20 | 627.778 | 0.001593 | 3.377 | 1.593 | 0.008569 |
| | | | | | | |
| 9 | 0 | 1059.333 | 0.000944 | 3.757 | 0.944 | 0.003348 |
| | 2.5 | 931 | 0.001074 | 4.39 | 1.074 | 0.005064 |
| | 5 | 817.56 | 0.001223 | 2.832 | 1.223 | 0.004236 |
| | 10 | 663.778 | 0.001507 | 3.864 | 1.507 | 0.008773 |
| | 15 | 563.989 | 0.001773 | 3.091 | 1.773 | 0.009717 |
| | 20 | 491.656 | 0.002034 | 2.793 | 2.034 | 0.011555 |
| | | | | | | |
| 17 | 0 | 966.333 | 0.001035 | 7.48 | 1.035 | 0.008012 |
| | 2.5 | 836.556 | 0.001195 | 9.001 | 1.195 | 0.012858 |
| | 5 | 738.222 | 0.001355 | 4.692 | 1.355 | 0.008612 |
| | 10 | 589.833 | 0.001695 | 2.628 | 1.695 | 0.007552 |
| | 15 | 498.579 | 0.002006 | 2.8 | 2.006 | 0.011266 |
| | 20 | 437.033 | 0.002288 | 2.62 | 2.288 | 0.013716 |
| | | | | | | |
| 23 | 0 | 876.111 | 0.001141 | 5.556 | 1.141 | 0.007236 |
| | 2.5 | 758.666 | 0.001318 | 4.285 | 1.318 | 0.007444 |
| | 5 | 658.383 | 0.001519 | 4.24 | 1.519 | 0.009782 |
| | 10 | 524.822 | 0.001905 | 2.697 | 1.905 | 0.00979 |
| | 15 | 443.044 | 0.002257 | 2.687 | 2.257 | 0.013688 |
| | 20 | 388.533 | 0.002574 | 2.268 | 2.574 | 0.015025 |
| | | | | | | |
| 29 | 0 | 10.178 | 0.001234 | 3.61 | 1.234 | 0.437683 |
| | 2.5 | 694.722 | 0.001439 | 4.322 | 1.439 | 0.008952 |
| | 5 | 603.211 | 0.001658 | 3.527 | 1.658 | 0.009694 |
| | 10 | 477.445 | 0.002094 | 2.832 | 2.094 | 0.012421 |
| | 15 | 404.356 | 0.002473 | 2.435 | 2.473 | 0.014892 |
| | 20 | 354.744 | 0.002819 | 1.969 | 2.819 | 0.015647 |

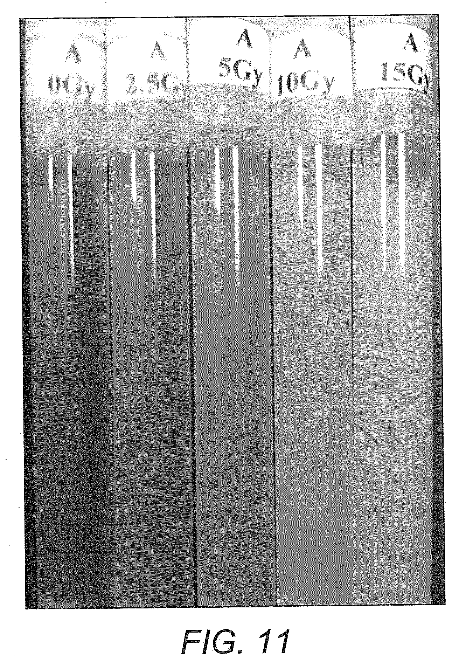

Fig. 10 shows dose response curves of NIBMAGAT (21, 22, 23, 24 and 25) polymer gel for different glycerol concentrations. The variation of absorbance versus absorbed dose for polymerization of NIBMAGAT for glycerol concentration from 0 to 29 wt% is shown in Fig. 10. The results show that the solubility of NIBMA within NIBMAGAT polymer gels increases with increasing glycerol concentration from 0 to 29 %. The dose response increases significantly with increase of glycerol concentration from 0 to 29 % in gel dosimeters. Upon irradiation, the color of the polymer gels changed to whitish along radiation beam as shown in Fig. 11, indicating polymerization process has taken place in the dosimeters due to crosslinking between the co-monomers. The degree of whiteness intensity is dependent on the increase of dose and could be analyzed using NMR method. Fig. 11 shows dose response of NIBMAGAT polymer gel for different glycerol concentrations in actual tubes.

### Effect of Dose Rate on NIBMAGAT Polymer Gel Dosimeter:

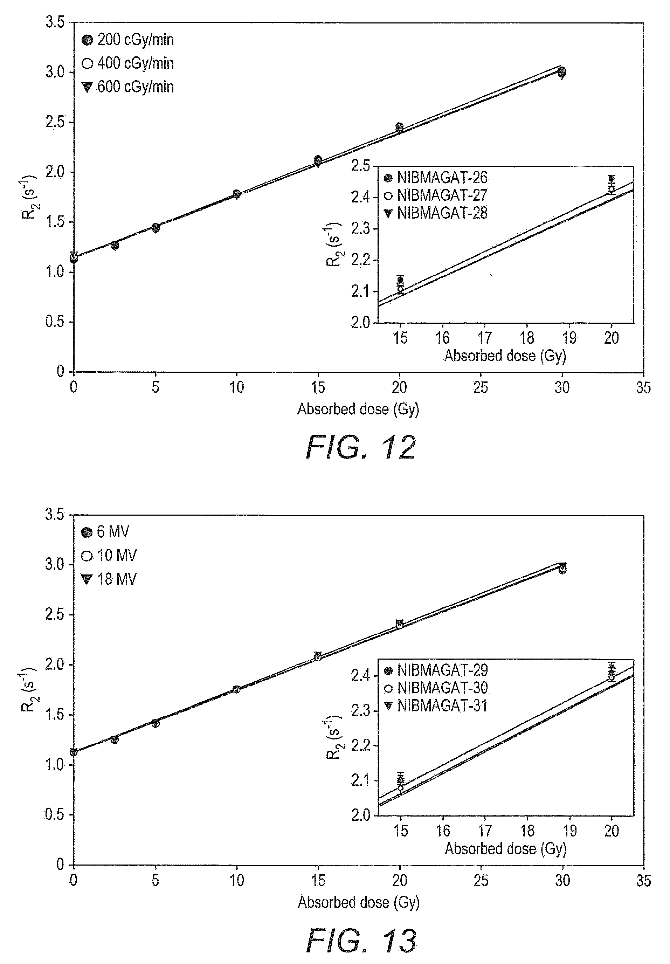

The effect of dose rate on the response of NIBMAGAT polymer gel dosimeters which its compositions based on 4 % gelatin, 1% NIBMA, 3% BisAAm, and 17% glycerol were investigated using 200, 400 and 600 cGy/minute under 10 MV radiation energy. The samples were irradiated for absorbed doses of 0, 2.5, 5, 10, 15, 20 and 30 Gy. Three dosimeters were irradiated at each dose point. A centigray (cGy) is a derived metric (SI) measurement unit of absorbed radiation dose of ionizing radiation, e.g. X-rays. The SI prefix 'centi' stands for one hundredths. The centigray is equal to one hundredth of a gray (0.01 Gy), and the gray is defined as the absorption of one joule of ionizing radiation by one kilogram (1 J/kg) of matter, e.g. human tissue. It was found that there is no appreciable effect of dose rate on NIBMAGAT polymer gel dosimeters as shown in Fig. 12. Fig. 12 shows dose response curves of NIBMAGAT (26, 27 and 28) polymer gel for different dose rate under 10 MV radiation energy. The results are also presented in Table 14 for further clarification.

**Table 14: NMR Relaxation Rate parameters of NIBMAGAT (26, 27 and 28) polymer gel for different dose rate under 10 MV radiation energy.**

| Dose Rate (cGy/ minute) | Dose, (Gy) | Relaxation Time (T₂), (ms) | Relaxation Rate (R₂ = 1/T₂), R₂, (ms⁻¹) | T₂ S.D. | Relaxation Rate (R₂ = 1/T₂), R₂,(s⁻¹) | R₂ S.D. |
|---|---|---|---|---|---|---|
| 200 | 0 | 891.889 | 0.001121 | 6.29 | 1.121 | 0.007906 |
| | 2.5 | 791.667 | 0.001263 | 2.64 | 1.263 | 0.004212 |
| | 5 | 690.222 | 0.001449 | 3.45 | 1.449 | 0.007243 |
| | 10 | 556.667 | 0.001796 | 3.54 | 1.796 | 0.011421 |
| | 15 | 466.97 | 0.002141 | 2.56 | 2.141 | 0.011737 |
| | 20 | 406.233 | 0.002462 | 2.18 | 2.462 | 0.013212 |
| | 30 | 331.022 | 0.003021 | 1.99 | 3.021 | 0.018161 |
| | | | | | | |
| 400 | 0 | 870.667 | 0.001149 | 5.51 | 1.149 | 0.007271 |
| | 2.5 | 787.889 | 0.001269 | 3.42 | 1.269 | 0.005508 |
| | 5 | 694.889 | 0.001439 | 3.887 | 1.439 | 0.008049 |
| | 10 | 561.445 | 0.001781 | 2.712 | 1.781 | 0.008603 |
| | 15 | 473.178 | 0.002113 | 3.017 | 2.113 | 0.013473 |
| | 20 | 411.011 | 0.002433 | 3.052 | 2.433 | 0.018066 |
| | 30 | 333.745 | 0.002996 | 2.044 | 2.996 | 0.018349 |
| | | | | | | |
| 600 | 0 | 847.778 | 0.00118 | 6.626 | 1.18 | 0.009223 |
| | 2.5 | 785.889 | 0.001272 | 3.376 | 1.272 | 0.005464 |
| | 5 | 693 | 0.001443 | 3.869 | 1.443 | 0.008056 |
| | 10 | 562.667 | 0.001777 | 3.1 | 1.777 | 0.00979 |
| | 15 | 474.144 | 0.002109 | 2.667 | 2.109 | 0.011863 |
| | 20 | 412.0447 | 0.002427 | 2.189 | 2.427 | 0.012894 |
| | 30 | 334.333 | 0.002991 | 2.024 | 2.991 | 0.018107 |

### Effect of Radiation Energy on NIBMAGAT Polymer Gel Dosimeter:

The effect of radiation energy on the response of NIBMAGAT polymer gel dosimeters which its compositions based on 4 % gelatin, 1% NIMA, 3% BIS, and 17% glycerol was investigated using 6, 10 and 18 MV at 600 Gy/ minute dose rate. The samples were irradiated for absorbed doses of 0, 2.5, 5, 10, 15, 20 and 30 Gy. Three dosimeters were irradiated at each dose point. It was found that there is no appreciable effect of radiation energy on NIBMAGAT polymer gel dosimeters as shown in Fig. 13. Fig. 13 Dose response curves of NIBMAGAT (29, 30 and 31) polymer gel for different radiation energies at 600 Gy/ minute dose rate. The results also presented in Table 15 for further clarification.

**Table 15: NMR Relaxation Rate parameters ofNIBMAGAT (29, 30 and 31) polymer gel for different radiation energies at 600 Gy/ minute dose rate.**

| Radiation energy (MV) | Dose, (Gy) | Relaxation Time (T₂), (ms) | Relaxation Rate (R₂ = 1/T₂), R₂, (ms⁻¹) | T₂ S.D. | Relaxation Rate (R₂ = 1/T2), R₂,(s⁻¹) | R₂ S.D. |
|---|---|---|---|---|---|---|
| | 0 | 894.111 | 0.001118 | 3.7 | 1.118 | 0.004626 |
| | 2.5 | 799.56 | 0.001251 | 3.42 | 1.251 | 0.005351 |
| 6 | 5 | 708.111 | 0.001412 | 3.85 | 1.412 | 0.007677 |
| | 10 | 568.222 | 0.00176 | 2.942 | 1.76 | 0.009112 |
| | 15 | 476.944 | 0.002097 | 2.22 | 2.097 | 0.009761 |
| | 20 | 414.979 | 0.00241 | 2.25 | 2.41 | 0.013067 |
| | 30 | 339.444 | 0.002946 | 1.947 | 2.946 | 0.016898 |
| | | | | | | |
| | 0 | 876.167 | 0.001141 | 7.56 | 1.141 | 0.009845 |
| | 2.5 | 792.333 | 0.001262 | 4.36 | 1.262 | 0.006944 |
| 10 | 5 | 708.444 | 0.001412 | 3.71 | 1.412 | 0.007394 |
| | 10 | 568.445 | 0.001759 | 2.98 | 1.759 | 0.009221 |
| | 15 | 481.789 | 0.002076 | 3.171 | 2.076 | 0.013664 |
| | 20 | 417.278 | 0.002396 | 1.863 | 2.396 | 0.010697 |
| | 30 | 335.889 | 0.002977 | 1.656 | 2.977 | 0.014677 |
| | | | | | | |
| | 0 | 874.222 | 0.001144 | 5.752 | 1.144 | 0.007527 |
| | 2.5 | 791.444 | 0.001264 | 5.522 | 1.264 | 0.008819 |
| 18 | 5 | 701.667 | 0.001425 | 3.291 | 1.425 | 0.006684 |
| | 10 | 564.222 | 0.001772 | 3.242 | 1.772 | 0.010182 |
| | 15 | 473.778 | 0.002111 | 2.483 | 2.111 | 0.011063 |
| | 20 | 411.656 | 0.002429 | 2.153 | 2.429 | 0.012704 |
| | 30 | 333.7 | 0.002997 | 2.08 | 2.997 | 0.018681 |

### Effect of Scanning Temperature:

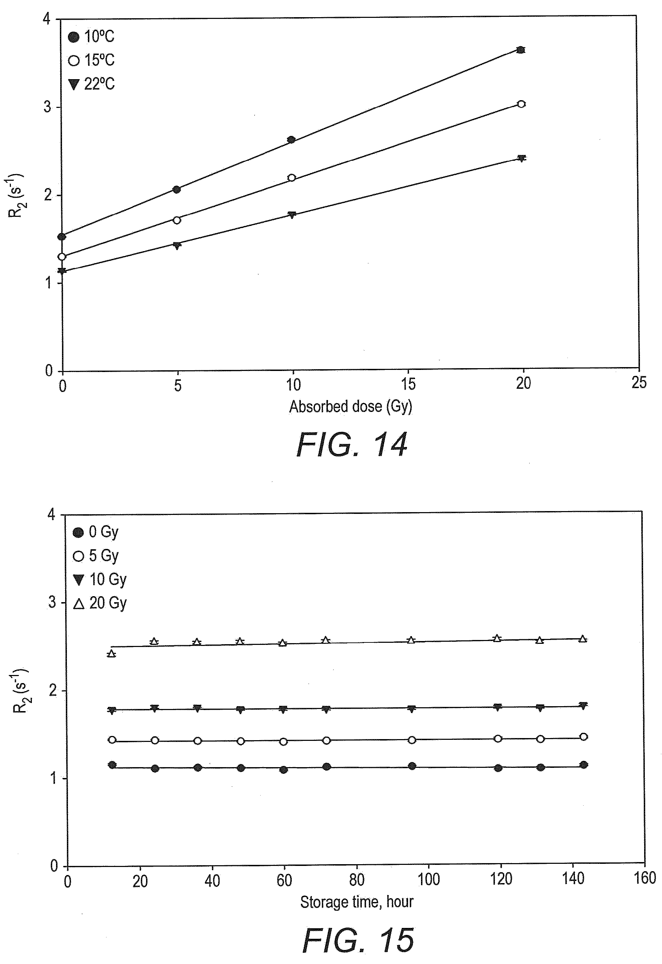

The effect of scanning temperature of NMR on relaxation rate R₂ of NIBMAGAT polymer gel dosimeters was investigated by irradiating samples of formulation based on 4 % gelatin, 1% NIMA, 3% BIS, and 17% glycerol to 5, 10 and 20 Gy. A set of three samples was used for each temperate. The results show that R₂ increases upon cooling the gel during the NMR measurement (Fig. 14) due to the slowdown of the segmental motions of the polymer chains which lead to an increase of the correlation times of the semi-solid protons. Therefore, the response of the dosimeters has to be corrected under actual processing conditions. Fig. 14 Dose response curves of NIBMAGAT (32, 33 and 34) polymer gel as a function of scanning temperature under 10 MV radiation energy at 600 Gy/ minute dose rate.

### Stability of NIBMAGAT Gel Dosimeters:

The stability of gel dosimeters was tested by irradiating NIBMAGAT dosimeters samples of formulations based on 4 % gelatin, 1% NIMA, 3% BIS, and 17% glycerol to 5, 10 and 20 Gy and storing in a refrigerator (10° C). A set of three samples was used for each dose. The change of the response of normoxic polymer gel was investigated with time after irradiation. The results (see Fig. 15) show no change in the relaxation rate or absorbance of the gel dosimeters up to 144 hours. Fig. 15 shows Dose response curves of NIBMAGAT (35, 36, 37 and 34) polymer gel as a function of storage time. Compared to Vandecasteele et. al. (2013) 3D gel dosimeter the stability of instant NIBMAGAT dosimeter after irradiation was very high up to 8 days.

### Dose Response of NIBMAGAT Gels:

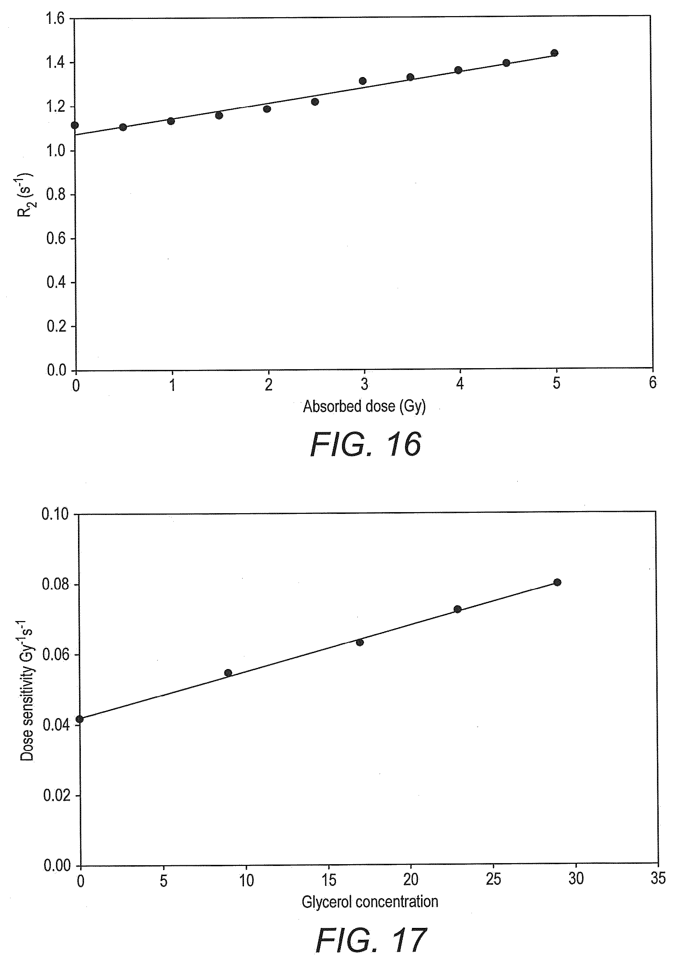

Dose response (R₂) at 24 hours post-irradiation for the gel dosimeter was investigated by preparing NIBMAGAT dosimeters of formulations based on 4 % gelatin, 1% NIMA, 3% BIS, and 17% glycerol irradiated up to 5 Gy by 0.5 Gy interval and storing in a refrigerator (10° C). A set of three samples was used for each dose. The change in the proton relaxation rate R₂ versus absorbed dose for polymerization of NIBMAGAT is shown in Fig. 16. The dose response of the gel increases gradually with increase of dose. As the dose increases, more radiation-induced free monomer radicals were generated due to the breakage of C=C bonds of the co-monomers, resulting in an increase of polymer gel formed. Linearity of dose response can be seen over the region up to 5 Gy. Fig. 16 Dose responses ofNIBMAGAT gels up to 5 Gy under 10 MV radiation energy at a dose rate of 600 Gy/ minute.

### Dose Sensitivity of NIBMAGAT Gel Dosimeters:

A relationship between glycerol concentration in the NIBMAGAT gel dosimeters and dose sensitivity has been observed with MR scanning. The dose sensitivity was taken from the slope of linear plot of dose D versus R₂ of Fig. 17. The results show that the sensitivity value increases gradually with increasing glycerol concentration. The dose sensitivity increased (based on the transverse relaxation rate R₂) by approximately 25% from concentrations from 0 to 30 %, leading to the increase of polymerization of NIBMAGAT gel dosimeters as shown in Fig. 9. The results show that glycerol based gels can significantly improve the dose sensitivity. Fig. 17 Dose sensitivity values of NIBMAGAT gel dosimeters for different glycerol concentration

### Reproducibility of NIBMAGAT Gels Preparation:

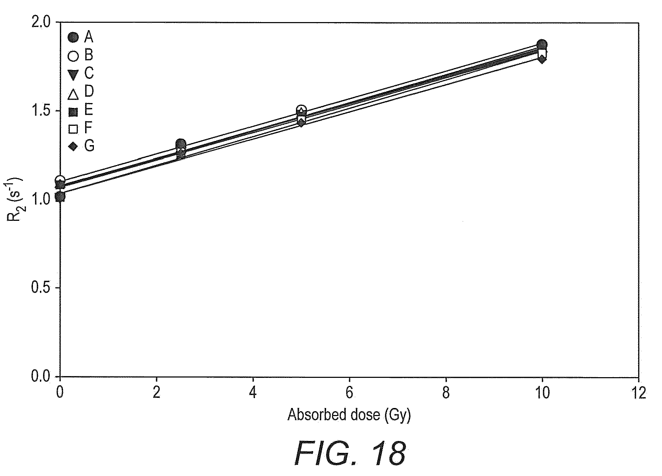

Fig. 18 presents the reproducibility of the 3 D gel dosimeters. The dose response (R₂) curves were obtained at 24 hours post-irradiation for the gel dosimeter was investigated by preparing NIBMAGAT dosimeters of formulations based on 4 % gelatin, 1% NIBMA, 3% BIS, and 17% glycerol irradiated up to 10 Gy. The results show no change in the dose response after reproducing the same dosimeter seven times. Fig. 18 shows dose response of NIBMAGAT gels up to 10 Gy for radiation energy of 10 MV at 600 Gy/minute dose rate reproduced 7 times.

In addition, it will be appreciated that the novel 3D polymer for dosimetric use disclosed herein may be embodied using means for achieving better quality images for medical use and diagnosis. Accordingly, the specification and drawings are to be regarded in an illustrative rather than a restrictive sense.

## Claims

1. A polymerizable composition (NIBMAGAT) for making a 3D gel dosimeter for planning a radiation treatment, the polymerizable composition comprising:
2-5 w/w% of gelatin;
1-2 w/w% of N-(isobutoxymethyl)acrylamide (NIBMA);
1-4 w/w% ofN,N-methylene-bis-acrylamide (BIS);
0-29 w/w% of at least one co-solvent selected from the group consisting of glycerol, acetone and methanol; and
ultra-pure de-ionized water as a solvent.

2. The composition of claim 1, further comprising:
1-20 mM, preferably 5 mM, of trakis(hydroxymethyl)phosphonium chloride (THPC).

3. The composition of any one of the previous claims, wherein the co-solvent is glycerol.

4. The composition of claim 3, comprising 17-20 w/w% of glycerol, preferably 20 w/w%.

5. The polymer composition of any one of the previous claims, comprising 4g w/w% of gelatin.

6. The composition of any of the previous claims, comprising 3-4 w/w% of N,N-methylene-bis-acrylamide (BIS), preferably 3 w/w%.

7. The composition of any of the previous claims, comprising 1-1.8 w/w% of N-(isobutoxymethyl)acrylamide (NIBMA), preferably 1 w/w%.

8. A method of making the polymerizable composition (NIBMAGAT) of any one of claims 1 to 7, comprising:
soaking gelatin for 10 minutes in ultra-pure deionized water to make a mixture of the gelatin and deionized water;
heating the mixture of the gelatin and the deionized water for 1 hour at 50 °C to make a gelatin solution;
cooling the gelatin solution to 40°C;
adding N,N-methylene-bis-acrylamide (BIS), N-(isobutoxymethyl)acrylamide (NIBMA) and at least on co-solvent selected from the group consisting of glycerol, acetone and methanol to the gelatin solution and mixing to make a second solution;
cooling the second solution to 35°C;
adding trakis(hydroxymethyl)phosphonium chloride (THPC) to the second solution to make the N-(isobutoxymethyl)acrylamide polymerizable composition (NIBMAGAT); and
storing the composition at 10°C in a refrigerator until its use for planning of radiation treatment.

9. The method of claim 8, wherein the steps of making the gelatin solution, the second solution and the N-(isobutoxymethyl)acrylamide polymerizable composition (NIBMAGAT) to be used for measuring 3D irradiation are performed at normal atmospheric conditions.

10. The method of claim 8, further comprising:
purging a chamber with nitrogen to remove oxygen; and
performing the steps of making the gelatin solution, the second solution and the N-(isobutoxymethyl)acrylamide polymerizable composition (NIBMAGAT) to be used for measuring 3D irradiation results within the purged chamber.

11. A method of using the polymerizable composition of any one of claims 1 to 7 for planning a radiation treatment, comprising:
making the polymerizable composition (NIBMAGAT) and pouring it into a tube;
storing the tube containing the polymerizable composition in a refrigerator at 10°C before use; and
irradiating the polymerizable composition using a linear accelerator at a specific absorbed dose to observe the dose response and plan radiation treatment for clinical use.

12. The method of claim 11, wherein the absorbed dose is between 0-20 Gy.

13. A 3D gel dosimeter comprising the polymerizable composition of any one of claims 1 to 7.

## Patentansprüche

1. Polymerisierbare Zusammensetzung (NIBMAGAT) zum Herstellen eines 3D-Geldosimeters zum Planen einer Strahlenbehandlung, wobei die polymerisierbare Zusammensetzung umfasst:
2-5 Gew.-% Gelatine;
1-2 Gew.-% N-(Isobutoxymethyl)acrylamid (NIBMA);
1-4 Gew.-% N,N-Methylenbisacrylamid (BIS);
0-29 Gew.-% wenigstens eines Hilfslösungsmittels, ausgewählt aus der Gruppe bestehend aus Glycerin, Aceton und Methanol; und
hochreines entionisiertes Wasser als Lösungsmittel.

2. Zusammensetzung nach Anspruch 1, weiterhin umfassend:
1-20 mM, vorzugsweise 5 mM, Tetrakis(hydroxymethyl)phosphoniumchlorid (THPC).

3. Zusammensetzung nach einem der vorangehenden Ansprüche, wobei das Hilfslösungsmittel Glycerin ist.

4. Zusammensetzung nach Anspruch 3, umfassend 17-20 Gew.-% Glycerin, vorzugsweise 20 Gew.-%.

5. Polymerzusammensetzung nach einem der vorangehenden Ansprüche, umfassend 4 Gew.-% Gelatine.

6. Zusammensetzung nach einem der vorangehenden Ansprüche, umfassend 3-4 Gew.-% N,N-Methylenbisacrylamid (BIS), vorzugsweise 3 Gew.-%.

7. Zusammensetzung nach einem der vorangehenden Ansprüche, umfassend 1-1,8 Gew.-% N-(Isobutoxymethyl)acrylamid (NIBMA), vorzugsweise 1 Gew.-%.

8. Verfahren zur Herstellung der polymerisierbaren Zusammensetzung (NIBMAGAT) nach einem der Ansprüche 1 bis 7, umfassend:
Einweichen von Gelatine in hochreinem entionisiertem Wasser für 10 Minuten, um ein Gemisch aus der Gelatine und dem entionisierten Wasser herzustellen;
Erhitzen des Gemischs aus der Gelatine und dem entionisierten Wasser für 1 Stunde bei 50°C, um eine Gelatinelösung herzustellen;
Abkühlen der Gelatinelösung auf 40 °C;
Zugeben von N,N-Methylenbisacrylamid (BIS), N-(Isobutoxymethyl)acrylamid (NIBMA) und wenigstens einem Hilfslösungsmittel, ausgewählt aus der Gruppe bestehend aus Glycerin, Aceton und Methanol, zu der Gelatinelösung und Mischen, um eine zweite Lösung herzustellen;
Abkühlen der zweiten Lösung auf 35 °C;
Zugeben von Tetrakis(hydroxymethyl)phosphoniumchlorid (THPC) zu der zweiten Lösung, um die polymerisierbare N-(isobutoxymethyl)acrylamid- Zusammensetzung (NIBMAGAT) herzustellen; und
Aufbewahren der Zusammensetzung bei 10°C in einem Kühlschrank bis zu ihrer Verwendung zum Planen einer Strahlenbehandlung.

9. Verfahren nach Anspruch 8, wobei die Schritte des Herstellens der Gelatinelösung, der zweiten Lösung und der polymerisierbaren N-(isobutoxymethyl)acrylamid-Zusammensetzung (NIBMAGAT), die zum Messen von 3D-Bestrahlung verwendet werden soll, bei normalen atmosphärischen Bedingungen durchgeführt werden.

10. Verfahren nach Anspruch 8, weiterhin umfassend:
Spülen einer Kammer mit Stickstoff, um Sauerstoff zu entfernen; und Durchführen der Schritte des Herstellens der Gelatinelösung, der zweiten Lösung und der polymerisierbaren N-(isobutoxymethyl)acrylamid- Zusammensetzung (NIBMAGAT), die zum Messen von 3D-Bestrahlungsergebnissen verwendet werden soll, in der gespülten Kammer.

11. Verfahren der Verwendung der polymerisierbaren Zusammensetzung nach einem der Ansprüche 1 bis 7 zum Planen einer Strahlenbehandlung, umfassend:
Herstellen der polymerisierbaren Zusammensetzung (NIBMAGAT) und Gießen derselben in eine Röhre;
Aufbewahren der Röhre, welche die polymerisierbare Zusammensetzung enthält, in einem Kühlschrank bei 10 °C vor der Verwendung; und
Bestrahlen der polymerisierbaren Zusammensetzung unter Verwendung eines Linearbeschleunigers mit einer spezifischen absorbierten Dosis, um die Dosiswirkung zu beobachten und eine Strahlenbehandlung für den klinischen Einsatz zu planen.

12. Verfahren nach Anspruch 11, wobei die absorbierte Dosis 0-20 Gy beträgt.

13. 3D-Geldosimeter, umfassend die polymerisierbare Zusammensetzung nach einem der Ansprüche 1 bis 7.

## Revendications

1. Composition polymérisable (NIBMAGAT) pour réaliser un dosimètre de gel 3D afin de planifier une radiothérapie, la composition polymérisable comprenant :
2-5 % pds/pds de gélatine ;
1-2 % pds/pds de N-(isobutoxyméthyl)acrylamide (NIBMA) ;
1-4 % pds/pds de N,N-méthylène-bis-acrylamide (BIS) ;
0-29 % pds/pds d'au moins un cosolvant sélectionné dans le groupe constitué de glycérol, d'acétone et de méthanol ; et
de l'eau déionisée ultra pure en tant que solvant.

2. Composition selon la revendication 1, comprenant également :
1-20 mM, de préférence 5 mM, de chlorure de tetrakis(hydroxyméthyl)phosphonium (THPC).

3. Composition selon l'une quelconque des revendications précédentes, dans laquelle le cosolvant est du glycérol.

4. Composition selon la revendication 3, comprenant 17-20 % pds/pds de glycérol, de préférence 20 % pds/pds.

5. Composition polymère selon l'une quelconque des revendications précédentes, comprenant 4 % pds/pds de gélatine.

6. Composition selon l'une quelconque des revendications précédentes, comprenant 3-4 % pds/pds de N,N-méthylène-bis-acrylamide (BIS), de préférence 3 % pds/pds.

7. Composition selon l'une quelconque des revendications
précédentes, comprenant 1-1,8 % pds/pds de N-(isobutoxyméthyl)acrylamide (NIBMA), de préférence 1 % pds/pds.

8. Procédé de production d'une composition polymérisable (NIBMAGAT) selon l'une quelconque des revendications 1 à 7, comprenant :
trempage de gélatine pendant 10 minutes dans de l'eau déionisée ultra pure pour produire un mélange de gélatine et d'eau déionisée ;
chauffage du mélange de gélatine et d'eau déionisée pendant 1 heure à 50 °C pour produire une solution de gélatine ;
refroidissement de la solution de gélatine jusqu'à 40 °C ;
ajout à la solution de gélatine de N,N-méthylène-bis-acrylamide (BIS), N-(isobutoxyméthyl)acrylamide (NIBMA) et d'au moins un cosolvant sélectionné dans le groupe constitué de glycérol, d'acétone et de méthanol et mixage pour produire une deuxième solution ;
refroidissement de la deuxième solution jusqu'à 35 °C ;
ajout de chlorure de tetrakis(hydroxyméthyl)phosphonium (THPC) à la deuxième solution pour produire la composition polymérisable N-(isobutoxyméthyl)acrylamide (NIBMAGAT ; et
entreposage de la composition à 10 °C dans un réfrigérateur jusqu'à son utilisation pour planifier une radiothérapie.

9. Procédé selon la revendication 8, dans lequel les étapes de production de la solution de gélatine, de la deuxième solution et de la composition polymérisable N-(isobutoxyméthyl)acrylamide (NIBMAGAT destinée à être utilisée pour mesurer l'irradiation 3D sont effectuées en présence de conditions atmosphériques normales.

10. Procédé selon la revendication 8, comprenant également :
la purge d'une chambre avec de l'azote pour enlever l'oxygène ; et
la réalisation des étapes destinées à produire la solution de gélatine, la deuxième solution et la composition polymérisable N-(isobutoxyméthyl)acrylamide (NIBMAGAT) destinée à être utilisée pour mesurer les résultats du rayonnement 3D à l'intérieur de la chambre purgée.

11. Procédé d'utilisation de la composition polymérisable selon l'une quelconque des revendications 1 à 7 pour planifier une radiothérapie, comprenant :
la production de la composition polymérisable (NIBMAGAT et le versement de celle-ci dans un tube ;
l'entreposage du tube contenant la composition polymérisable dans un réfrigérateur à 10 °C avant utilisation ; et
l'irradiation de la composition polymérisable à l'aide d'un accélérateur linéaire selon une dose absorbée spécifique pour observer la relation dose-effet et planifier une radiothérapie destinée à une utilisation clinique.

12. Procédé selon la revendication 11, dans lequel la dose absorbée est 0-20 Gy.

13. Dosimètre de gel 3D comprenant la composition polymérisable selon l'une quelconque des revendications 1 à 7.
